(19) Europäisches Patentamt
European Patent Office
Office européen des brevets

(11) **EP 3 871 748 A1**

(12) **EUROPEAN PATENT APPLICATION**
published in accordance with Art. 153(4) EPC

(43) Date of publication:
**01.09.2021 Bulletin 2021/35**

(21) Application number: **19876978.8**

(22) Date of filing: **21.10.2019**

(51) Int Cl.:
**B01D 39/08** (2006.01)    **B01D 39/16** (2006.01)
**B01D 39/20** (2006.01)    **B03B 5/00** (2006.01)
**B07B 1/00** (2006.01)    **B07B 1/46** (2006.01)
**C12M 1/00** (2006.01)    **C12M 3/06** (2006.01)

(86) International application number:
**PCT/JP2019/041306**

(87) International publication number:
**WO 2020/085301 (30.04.2020 Gazette 2020/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.10.2018 JP 2018199182**

(71) Applicants:
• **Teijin Limited**
  **Osaka 530-0005 (JP)**
• **JCR Pharmaceuticals Co., Ltd.**
  **Ashiya-shi, Hyogo 659-0021 (JP)**

(72) Inventors:
• **KOHNO, Azusa**
  **Osaka-shi, Osaka 530-0005 (JP)**
• **KUSHIDA, Takashi**
  **Osaka-shi, Osaka 530-0005 (JP)**
• **ISHIWARI, Ayumi**
  **Osaka-shi, Osaka 530-0005 (JP)**
• **IMAGAWA, Kiwamu**
  **Kobe-shi, Hyogo 651-2241 (JP)**
• **HOSODA, Yuki**
  **Kobe-shi, Hyogo 651-2241 (JP)**

(74) Representative: **Cockerton, Bruce Roger**
  **Carpmaels & Ransford LLP**
  **One Southampton Row**
  **London WC1B 5HA (GB)**

(54) **FILTER FOR FILTERING, CONTAINER WITH FILTER, AND METHOD FOR REMOVING FOREIGN MATTER IN CELL SUSPENSION**

(57)    A filtration filter is configured from a first weld frame, a second weld frame, and a filter sandwiched and welded between the first weld frame and the second weld frame. The first weld frame and the second weld frame are configured by a flexible film having a film thickness of at least 120 μm. The filter is configured by a substance having a higher melting point than the first weld frame and the second weld frame and having an opening area ratio of from 10% to 80%. The first weld frame is configured by a polymer that includes either a high density polyethylene having a melting point of from 120°C to 140°C, a linear low density polyethylene having a melting point of from 105°C to 125°C, or a mixture including at least one of the high density polyethylene or the linear low density polyethylene.

FIG.2

## Description

Technical Field

Technology disclosed herein relates to a filtration filter, a filter-equipped container, and a method for removing foreign matter from a cell suspension.

Background Art

A focus of recent interest is directed toward cell therapy and regeneration therapy to treat diseases by sampling cells from a body fluid or tissue from a patient himself or from a donor, culturing the sampled cells, and directly transplanting the cells obtained thereby into the affected area, or using the cells to seed a scaffold material which is then transplanted into the affected area. Cell therapy and regeneration therapy are actually being performed using some types of tissue such as skin and cornea, bone, cartilage and the like, and this gives rise to great expectations for next-generation therapeutic methods.

When performing cell therapy and regeneration therapy, the cells sampled from the patient need to be cultured and propagated in order to secure a given number of cells. The propagated cells are recovered from a culture solution after the given number of cells is obtained, and the cells are washed and concentrated so as to enable the cells to be used in cell therapy and regeneration therapy.

However, culture medium, blood serum, carriers used in culturing, waste products and debris derived from cells, and the like are contained in the cell suspension after culture, and so there is a need to separate and remove these components. There is furthermore a need to also wash and concentrate the cells after these components have been removed, and a known method to perform this is a centrifugal separation method. For example, International Publication WO2013/114845 proposes a cell culture kit. The kit is configured by a closed environment in which a culture container for culturing cells, a culture medium storage container for storing culture medium and the like, a cell injector container for injecting cells, and a cell recovery container for recovering a cell suspension after culture, are connected together by tubing. Such a cell culture kit enables processes from injecting cells, through the addition of a culture medium, sampling, and recovery to be performed within the kit while maintaining a closed environment.

The International Publication WO2013/114845 also illustrates an example in which, when recovering cultured cells, after leaving the culture container to stand and letting cells in the cell culture solution precipitate, the supernatant from the cell culture solution is discharged, and the cell culture solution now concentrated by the reduction in liquid volume is transferred from the culture container to the cell recovery container.

However, in order to recover cells in this manner, before discharging the supernatant from the cell culture solution, the culture container first needs to be left to stand and the cells in the cell suspension left to precipitate, and so time is accordingly required until the cells are precipitated. Moreover, even when sufficient time is taken to precipitate out the cells in the cell culture solution, there is still a concern that cells might become mixed in with the supernatant by handling during discharge, and that cells might be discharged together with the supernatant.

WO No. 2014/007382 describes causing layers of melted resin to intrude into openings in a metal filter, and welding opposing resin layers together using laser welding so as to achieve sufficient weld strength. There is, however, an issue of the poor flexibility of a metal filter when employed for processing cells in a closed environment.

Japanese Patent Application JP2006-231875A describes a vehicle fluid filter employing polyester resin fibers (non-woven fabric) as a filter, with the filter sandwiched between case configuration members. JP2006-231875A describes the use of uncolored NYLON 66, NYLON 6, etc. without pigment or the like as the resin for one case configuration member, and the use of colored NYLON 66, NYLON 6, etc. colored with a pigment or the like as the resin for the other case configuration member.

SUMMARY OF INVENTION

Technical Problem

An object of the technology disclosed herein is to provide a filtration filter having a filter fixed with high weld strength between two polymer films, to provide a filter-equipped container having a filtration filter welded so as to section the interior thereof, and to provide a method of removing foreign matter from a cell suspension by using a filter-equipped container.

Solution to Problem

The inventors of technology disclosed herein have discovered materials and film thicknesses of polymer films and an opening area ratio of a filter to obtain a high weld strength between these polymer films and filter when producing a filtration filter having a filter fixed between two polymer films, and accordingly completed the technology disclosed herein based on this knowledge.

Namely, the technology disclosed herein is a filtration filter including, for example, a first weld frame, a second weld frame, and a filter. The first weld frame is configured by a flexible polymer film that includes a polymer and that has a film thickness of at least 120 μm, the first weld frame being formed into a frame shape including a first through hole penetrating an inside of the frame shape in a thickness direction. The second weld frame is configured by a flexible polymer film that includes a polymer and that has a film thickness of at least 120 μm, the second weld frame being formed into a frame shape including a second through hole penetrating an inside of the frame shape in the thickness direction. The filter is configured by a substance having a higher melting point than the substances of these polymer films and is provided with openings and has an opening area ratio due to the openings of from 10% to 80%. The filter is welded to both the first weld frame and the second weld frame in a state in which an outer peripheral portion of the filter is sandwiched between an entire periphery of the first weld frame and an entire periphery of the second weld frame. The first weld frame is configured by polymer including either a high density polyethylene (HDPE) having a melting point of from 120°C to 140°C, a linear low density polyethylene (LLDPE) having a melting point of from 105°C to 125°C, or a mixture including at least one of the high density polyethylene or the linear low density polyethylene.

Reference here to "opening area ratio" means a proportion of surface area of openings with respect to overall surface area.

The technology disclosed herein also includes a filter-equipped container having such a filtration filter welded to an interior of a container made from a polymer, so as to section the interior of the container thereby.

Moreover, an aspect of the technology disclosed herein relates to a method of removing foreign matter from a cell suspension by injecting a cell suspension containing foreign matter or that might contain foreign matter into one section of the interior of a container in such a filter-equipped container, passing the cell suspension through the filtration filter, and recovering a filtrate containing cells from another section. The filter includes openings communicating through in a thickness direction. These openings have a diameter that enables cells to be passed through, but does not let foreign matter of a certain size or greater to pass through. Such foreign matter can accordingly be removed from a cell suspension by passing the cell suspension through the filtration filter.

Furthermore, an aspect of the technology disclosed herein relates to a method of removing foreign matter from a cell suspension. This method includes injecting a cell suspension into the one section of the interior of the container in such a filter-equipped container, passing the cell suspension through the filtration filter, recovering a filtrate containing cells, and also injecting liquid for suspending cells into the same section, re-suspending cells remaining in the same section, passing the re-suspended cell suspension through the filtration filter, and recovering a filtrate containing cells.

Advantageous Effects

In the filtration filter and filter-equipped container of technology disclosed herein, portions of the filter not sandwiched between the first weld frame and the second weld frame, namely portions corresponding the first through hole and the second through hole, exhibit a function as a filter. Due to the filter being welded to the first weld frame and the second weld frame with a high weld strength, the weld portion does not easily separate, and there is little concern that fluid might leak from the weld portion.

Thus there is little concern of solution leakage from the weld portion in cases in which the filter-equipped container of technology disclosed herein is employed to execute the method to remove foreign matter from a cell suspension of technology disclosed herein.

BRIEF DESCRIPTION OF DRAWINGS

Fig. 1 is a plan view illustrating a filter-equipped container according to an exemplary embodiment.
Fig. 2 is an exploded perspective view illustrating a filter-equipped container according to the exemplary embodiment.
Fig. 3 is schematic perspective view illustrating a filter-equipped container according to the exemplary embodiment.
Fig. 4 is a cross-section sectioned along line A-A of Fig. 1.

DESCRIPTION OF EMBODIMENTS

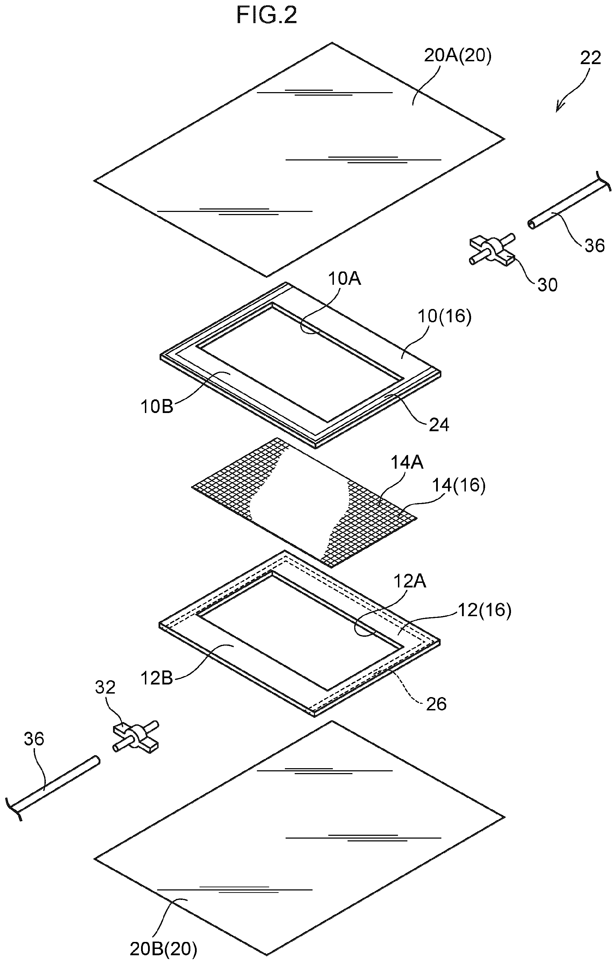

As in the example illustrated in Fig. 2, an exemplary embodiment of technology disclosed herein is a filtration filter 16. The filtration filter 16 includes a first weld frame 10 and a second weld frame 12, and a planar shaped filter 14 sandwiched between the first weld frame 10 and the second weld frame 12 and fixed thereto by welding. The filter 14 is made from a substance having a higher melting point than the substance of the first weld frame 10 and the second weld frame 12 and includes a portion not sandwiched between the first weld frame 10 and the second weld frame 12.

Detailed explanation follows regarding each member configuring a filtration filter of technology disclosed herein, using the filtration filter 16 illustrated in Fig. 2 as an example. The substance of the filter 14 preferably includes at least one type of substance selected from the group consisting of: polyolefin such as polypropylene, polyethylene, or the like; polyester; polyvinylchloride; polyvinyl alcohol; vinylidene chloride; acrylic polymer such as polymethyl methacrylate, polyacrylonitrile, or the like; polyamide such as NYLON; polystyrene; polyurethane; polyimide; aramid; polyether ether ketone; polysulfone; RAYON; cellulose; chitin; chitosan; cotton; hemp; glass; carbon fiber; and metal. Among these substances, the substance of the filter 14 preferably includes at least one type of substance selected from the group consisting of polyester, polyamide, polyolefin, polyether ether ketone, polyether sulfone, carbon fiber, and metal. The substance of the filter 14 more preferably includes at least one type of substance selected from the group consisting of polyester, polyamide, polyolefin, polyether ether ketone, polyether sulfone, carbon fiber, and metal. Most preferably the substance of the filter 14 includes at least polyester or polyamide.

Specific examples of polyesters include polyethylene terephthalate, polybutylene terephthalate, and polymethylene terephthalate. Specific examples of polyamides include NYLON 66, NYLON 6, NYLON 12, and the like.

The substance of the filter 14 may be a porous body form with a communicating-hole structure including plural openings 14A, an aggregate of fibers, a non-woven fabric, a woven product, a knitted product, or the like, the woven product or the knitted product is preferable.

The diameter of the openings 14A of the filter 14 is a size needed to trap impurities other than cells, such as culture carrier residue, and is preferably from 5 $\mu$m to 200 $\mu$m. Concerns arise that blockage of the filter 14 might arise and the impurity removal efficiency might fall when the diameter of the openings 14A is smaller than 5 $\mu$m. On the other hand, trapping impurities and target cells becomes difficult when the diameter of the openings 14A is larger than 200 $\mu$m. The diameter of the openings 14A is preferably from 10 $\mu$m to 200 $\mu$m in light of efficiency to remove impurities such as relatively large culture carrier residue and ability to trap target cells.

The opening area ratio of the filter 14 is preferably from 10% to 80% in consideration of in light of weld strength. Having an opening area ratio lower than 10% makes it difficult to entangle melted polymer film with the filter 14 when the polymer films configuring the first weld frame 10 and the second weld frame 12 are being welded, resulting in an issue that inter-layer separation or the like is liable to occur. On the other hand, having an opening area ratio of the filter 14 higher than 80% reduces the strength of the filter 14, with the possibility of cuts and breaks or cracks occurring, as well as a concern regarding a reduction in the mechanical strength as the filtration filter 16. The opening area ratio of the filter 14 is preferably from 10% to 70%, and is more preferably from 10% to 50%.

Moreover, the substance of the filter 14 has a higher melting point than the substances of the first weld frame 10 and the second weld frame 12. The substance of the filter 14 preferably has a melting point in a range of from 80°C to 180°C higher than the melting point of the substances of the first weld frame 10 and the second weld frame 12, more preferably has a melting point in a range of from 100°C to 160°C higher, and even more preferably has a melting point in a range of from 110°C to 150°C higher. For example, in cases in which the substances of the first weld frame 10 and the second weld frame 12 are both high density polyethylene having a melting point from 120°C to 140°C, the melting point of the substance of the filter 14 is preferably from 200°C to 320°C, is more preferably from 220°C to 300°C, and is even more preferably from 230°C to 290°C. Moreover, for example, in cases in which the substances of the first weld frame 10 and the second weld frame 12 are both linear low density polyethylene having a melting point from 105°C to 125°C, the melting point of the substance of the filter 14 is preferably from 185°C to 305°C, is more preferably from 205°C to 285°C, and is even more preferably from 215°C to 275°C.

Moreover, the film thickness of the filter 14 is preferably from 50 $\mu$m to 200 $\mu$m in light of the strength of the filter 14 after welding. There is a reduction in the strength of the filter 14 when the film thickness of the filter 14 is less than 50 $\mu$m, with the possibility of cuts and breaks or cracks occurring, as well as there being a concern that a reduction might occur in the mechanical strength of the filtration filter 16. On the other hand, when the film thickness of the filter 14 is more than 200 $\mu$m, there is concern that, when the polymer films configuring first weld frame 10 and the second weld frame 12 have been melted, there might be insufficient weld strength due to polymer film material only being melted at the openings 14A of the filter 14 in a region close to the surface of the filter 14 and not penetrating therein, making it difficult to instigate welding between the opposing first weld frame 10 and the second weld frame 12 etc.

The substance of the first weld frame 10 and the second weld frame 12 is preferably a single substance or a mixture of plural substances selected from the group consisting of: polyolefin such as polypropylene, polyethylene, high density polyethylene, low density polyethylene, linear low density polyethylene, ethylene-propylene copolymer or the like; polyester; polyvinylchloride; polyvinyl alcohol; vinylidene chloride; polystyrene; acrylic polymer such as polymethyl methacrylate and polyacrylonitrile, or the like; polyamide; polyurethane; polyimide; an aramid; polyether ether ketone; polysulfone; and carbon. More preferable therefrom is a single substance or a mixture of plural substances selected from the group consisting of polyolefin such as polypropylene, polyethylene, high density polyethylene, low density polyethylene, linear low density polyethylene or the like; polyester; polyamide; and polyvinyl alcohol. Most preferable therefrom is high density polyethylene or linear low density polyethylene. In particular in cases in which the substance is high density polyethylene, the melting point thereof is preferably in the range of from 120°C to 140°C, and in cases in which the substance is linear low density polyethylene the melting point thereof is preferably in the range of from 105°C to 125°C.

There are no particular limitations to combinations for the mixtures of two or more types of substance. Preferable combinations therefrom include either polyethylene and polyamide, or polyethylene and polyvinyl alcohol.

When employed as the substance for the first weld frame 10 and the second weld frame 12, the density of high density polyethylene having a melting point in the range of from 120°C to 140°C is from about 910 kg/m$^3$ to about 950 kg/m$^3$, and the density of linear low density polyethylene having a melting point in the range of from 105°C to 125°C is from about 912 kg/m$^3$ to about 930 kg/m$^3$.

Note that the melting point of the substances listed above is obtained as a temperature of an endothermic peak position as measured using differential scanning calorimetry (DSC). The densities of the substances mentioned above are obtained by measuring using a buoyancy weighing method as described in JIS K 0061 "Density and specific weight measurement method for chemical products" and in JIS Z 8807 "Density and specific weight measurement method for solids".

For the filtration filter 16 of technology disclosed herein, the first weld frame 10 and the second weld frame 12 both preferably have a frame shape with a first through hole 10A or a second through hole 12A penetrating through at the inside of the frame in the thickness direction. The first weld frame 10, the filter 14, and the second weld frame 12 are also preferably welded together welded together in a state in which a fluid in the filtration target is able to move in the sequence through (i) the first through hole 10A inside the frame of the first weld frame 10, (ii) parts of the filter 14 not contacting either frame portions 10B, 12B of the first weld frame 10 and the second weld frame 12, and (iii) the second through hole 12A inside the frame of the second weld frame 12, or is able to move through in the opposite sequence thereto.

In such cases in which both the first weld frame 10 and the second weld frame 12 are frame shaped, they may both be identical or different from to each other. However, the first through hole 10A that is the punched-out portion of the first weld frame 10 and the second through hole 12A that is the punched-out portion of the second weld frame 12 are preferably the same shape as each other, and rectangular shapes (including square shapes) are preferable therefor. Moreover, the first weld frame 10 and the second weld frame 12 both preferably have similar shapes for the external profiles of the first weld frame 10 and the second weld frame 12 (with the widths of the frame portions 10B, 12B being substantially the same in such cases). In particular, preferably the first through hole 10A of the first weld frame 10 and the second through hole 12A of the second weld frame 12 are both the same rectangular shape, and the external profiles of the first weld frame 10 and the second weld frame 12 are similar rectangular shapes. Most preferable is for the external profile of the first weld frame 10 and the external profile of the second weld frame 12 to be the same rectangular profile.

In the planar shaped filter 14 of technology disclosed herein, the functionality of the filter 14 is exhibited by the portion not sandwiched by the first weld frame 10 and the second weld frame 12, namely the portion corresponding to the first through hole 10A of the first weld frame 10 and the second through hole 12A of the second weld frame 12. In cases in which both the first weld frame 10 and the second weld frame 12 are frame shaped in the filtration filter 16 of technology disclosed herein, fluid in the filtration target moves sequentially through the first through hole 10A inside the frame of the first weld frame 10, then through the portion of the filter 14 corresponding to the first through hole 10A of the first weld frame 10 and the second through hole 12A of the second weld frame 12, then through the second through hole 12A inside the frame of the second weld frame 12, or in the opposite sequence thereto, as described above, but movement of the fluid is blocked by portions where the first weld frame 10 and the second weld frame 12 are welded.

Thus in cases in which both the first weld frame 10 and the second weld frame 12 are frame shaped in the filtration filter 16 of technology disclosed herein, in order for the filter 14 to exhibit a function as a filter in the filtration filter 16 of technology disclosed herein, the external profile of the filter 14 should be such that an overlapping portion is formed between the first weld frame 10 and the second weld frame 12 around the entire periphery of the first through hole 10A of the first weld frame 10 and the second through hole 12A of the second weld frame 12.

The shape of the filter 14 employed in the technology disclosed herein is preferably a rectangular shape (including a square shape). In such cases the first weld frame 10 and the second weld frame 12 preferably have the same overlapping shape at a peripheral edge portion of the thus shaped filter 14.

The film thicknesses of the first weld frame 10 and the second weld frame 12 are both at least 120 μm due to the need to obtain a high weld strength to the filter 14. The upper limit to the film thicknesses of the first weld frame 10 and the second weld frame 12 is not particularly limited in light of weld strength to the filter 14, however a film thickness not exceeding 500 μm is preferable in light of imparting flexibility thereto. The film thicknesses of the first weld frame 10 and the second weld frame 12 are preferably from 200 μm to 400 μm, and are most preferably from 200 μm to 300 μm.

The weld strength of a weld portion 34 may be measured by the method described in Example B (weld strength evaluation method), and is preferably at least 20 N/15 mm, more preferably at least 23N/15 mm, and most preferably at least 30 N/15 mm. For example, the weld strength of the weld portion 34 is from 20 N/15 mm to 80 N/15 mm, is from 23N/15 mm to 60 N/15 mm, or is from 30N/15 mm to 60 N/15 mm. Note that a standard value in JIS Z 0238 is 23N/15 mm, and the filtration filter 16 of the technology disclosed herein may also employ this as a standard to the numerical value to achieve for the weld strength of the weld portion 34. However this does not mean that a weld strength of the weld portion 34 falling below this numerical value is immediately unusable.

The filtration filter 16 of the technology disclosed herein is what is referred to as a multilayered body including three layers configured by sandwiching the filter 14 between the first weld frame 10 and the second weld frame 12, heating these to a temperature in a range from the melting point of the first weld frame 10 or the second weld frame 12 to the melting point of the filter 14, so as to cause the polymer of the melted first weld frame 10 and second weld frame 12 to intrude into the openings 14A of the unmelted filter 14, before the polymer is then solidified by heat dissipation, for example, so as to fix the filter 14 to both the first weld frame 10 and the second weld frame 12 through the weld portion 34. In particular, melted polymer from both surfaces of the filter 14 preferably passes through the openings 14A of the unmelted filter 14 and connects, so that the filter 14 is fixed by the opposing first weld frame 10 and second weld frame 12 being joined together.

In the filtration filter 16 of technology disclosed herein, at least one of the first weld frame 10 or the second weld frame 12 may be welded to another member. In such cases at least one out of the first weld frame 10 or the second weld frame 12 in the technology disclosed herein functions as a medium to realize welding to the other member.

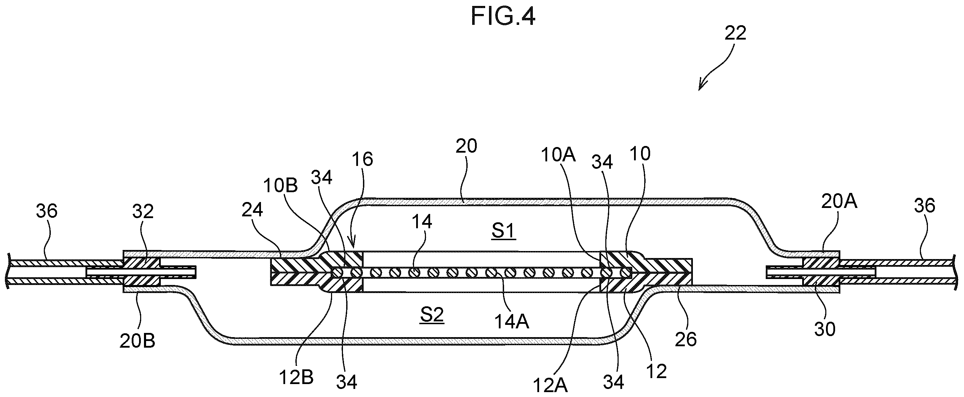

As illustrated in the example of Fig. 4, an exemplary embodiment of the technology disclosed herein is a filter-equipped container 22 in which the filtration filter 16 is welded to an interior of a container 20 that includes two sheets of polymer (20A, 20B), such that the interior of the container 20 is sectioned by the filtration filter 16. Detailed explanation follows regarding a filter-equipped container of technology disclosed herein, with reference to the example of the filter-equipped container 22 illustrated in Fig. 4.

Reference to "sectioned" means that, as illustrated in Fig. 4, the interior of the container 20 is partitioned by the filtration filter 16 in a state in which a flow path for a fluid to move from a section S1, which is a space configured between one face of the filtration filter 16 and the interior of the container 20, to a section S2, which is a space configured between the other face of the filtration filter 16 and the interior of the container 20 is not present other than at the filter 14 of the filtration filter 16. Adopting such a configuration results in the filter-equipped container 22 of technology disclosed herein functioning as a filter module.

The container 20 of technology disclosed herein includes the two polymer sheets (20A, 20B). The substance employed for these polymer sheets is a flexible plastic, and specific examples thereof are the same as those previously listed for the second weld frame 12 of technology disclosed herein. Preferable examples thereof also having similar physical properties (and in particular melting point) thereto, and in particular a substance preferably employed has compatibility to the substance of the first weld frame 10 and the substance of the second weld frame 12, because a welding method can be employed when fixing the filtration filter 16 of technology disclosed herein to the interior of the container 20. For example, in cases in which the substances of the first weld frame and the second weld frame and the flexible plastic are high density polyethylene having a melting point from 120°C to 140°C, the same high density polyethylene can also be suitably employed as the substance of the flexible plastic. Moreover, for example, in cases in which the substances of the first weld frame and the second weld frame are linear low density polyethylene having a melting point from 105°C to 125°C, the same linear low density polyethylene can also be suitably employed as the substance of the flexible plastic.

A preferable example of the shape of the container 20 is a shape produced by two opposing sheets of polymer having the same shape, for example a rectangular shape, and welding these together at a peripheral edge portion thereof.

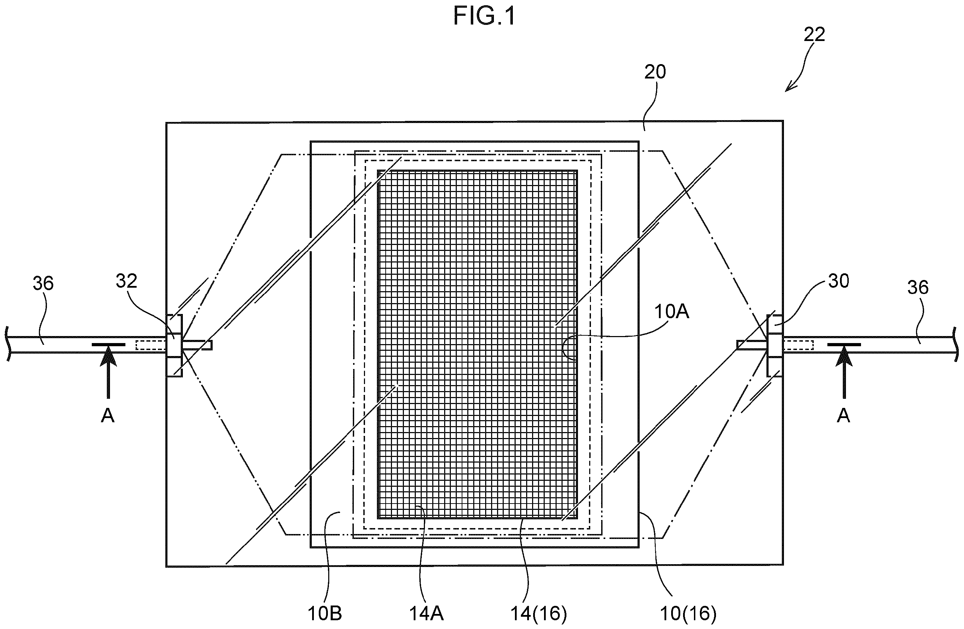

A preferable configuration adopted in such cases is one in which the filtration filter 16 is a substantially similar shape but slightly smaller than the rectangular shape of the container 20 (see Fig. 1), the first weld frame 10 is welded to one inside face of the container 20 through a first weld portion 24, and the second weld frame 12 is welded to the other inside face of the container 20 through a second weld portion 26. Note that the materials for the polymer "sheets" configuring the container 20 and for the polymer "films" configuring the first weld frame 10 and the second weld frame 12 are materials having substantially the same with flexibility. However, the explanation will discriminate these as either "sheet" or "film" to facilitate understanding of the position where they are employed in the present exemplary embodiment.

The first weld portion 24 is, as illustrated in Fig. 2, formed on one of the polymer sheets of the container 20 in a substantially U-shape that is open in one direction (at a length direction one end portion side of the container 20 in the present exemplary embodiment) as viewed in a thickness direction of the first weld frame 10. Moreover, the second weld portion 26 is formed on the other polymer sheet of the container 20 in a substantially U-shape that is open in another direction (at the other one end portion side in the length direction of the container 20 in the present exemplary embodiment) as viewed in the thickness direction of the second weld frame 12. Namely, the first weld portion 24 and the second weld portion 26 have open positions disposed at opposite sides.

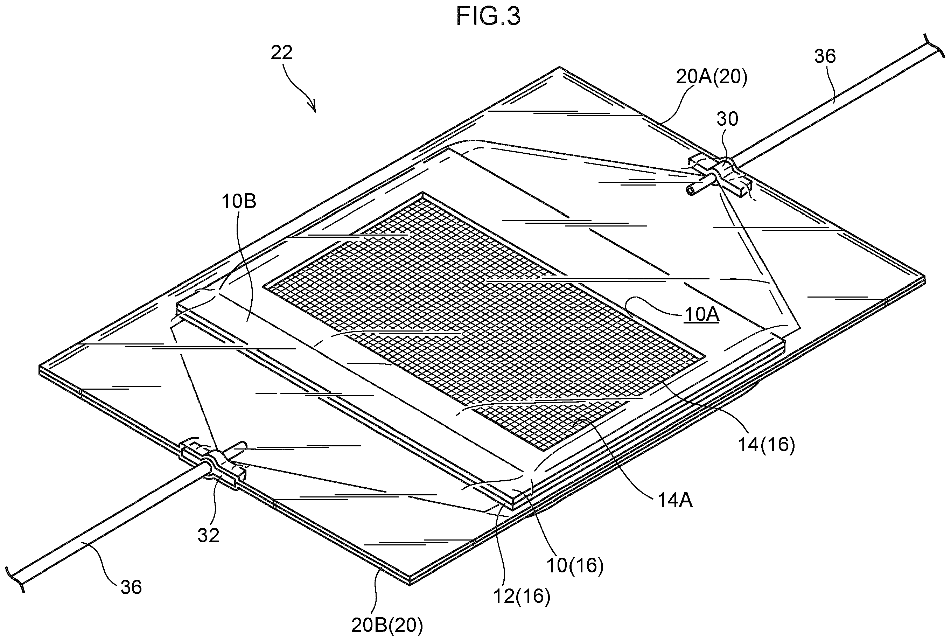

Furthermore, as illustrated in Fig. 3, the container 20 employed in the technology disclosed herein is preferably provided with at least one out of an injection port 30 or a discharge port 32. Particularly preferable is a configuration in which the injection port 30 is provided at one section inside the container 20 sectioned by the filtration filter 16 of technology disclosed herein, and the discharge port 32 is provided at the other section thereof. A configuration may be adopted in which there are plural injection ports 30 and plural discharge ports 32 provided with, for example, with both of the injection port 30 and the discharge port 32 provided at both sections. An injection port includes a pathway to connect an inside and an outside of a container together, and is a port employed for injecting the cell suspension into the container 20. Other than this pathway, there is no other pathway to enable the cell suspension to be injected inside the container 20 of the filter-equipped container 22. A discharge port, on the other hand, is for discharging the cell suspension from inside the container 20. Other than this pathway, there is no other pathway to enable the cell suspension inside the container 20 of the filter-equipped container 22 to be discharged. Namely, the polymer sheet 20A and the polymer sheet 20B are welded together except for at sites where the first weld portion 24 and the second weld portion 26 are open at the locations where the injection port and the discharge port are attached. Moreover, the discharge port and the injection port are welded or bonded to the polymer sheet 20A and to the polymer sheet 20B such that the inside of the container 20 is hermetically sealed, other than at the pathways where the ports are provided.

Furthermore, the filter-equipped container 22 of technology disclosed herein may be configured such that the interior of the container 20 is sectioned by n individual filtration filters 16 into n+1 spaces (wherein n is a natural number of 2 or more). In such cases some or all of the sections in the interior of the container 20 may be provided with at least one of the injection port 30 or the discharge port 32 able to connect to the inside of each interior section.

Moreover, the technology disclosed herein includes a method to remove foreign matter from a cell suspension that is a method including a process to inject a non-illustrated cell suspension into the one section S1 of the interior of the container 20 in the filter-equipped container 22 of technology disclosed herein, as illustrated in Fig. 4, and to recover the cell suspension from which foreign matter has been removed from the other section S2. Foreign matter such as, for example, redundant culture carrier, cell agglomerations, and the like, remains in the section S1 that the cell suspension had been injected into.

Furthermore, the technology disclosed herein includes a method to remove foreign matter from a cell suspension that is a method including a process to inject a cell suspension into the one section S1 of the interior of the container 20 in the filter-equipped container 22 of technology disclosed herein, to filter cells using the filter 14, to inject a liquid used for re-suspending cells into the same section S1, to re-suspend cells, and to recover cell suspension from the same section S1.

These methods, i.e. the method for removing foreign matter from a cell suspension and the method to remove foreign matter mixed with cells in a cell suspension, need an operation to separate cell suspension components using the filter 14, and examples of means employed to drive this operation include using the weight of the cell suspension itself, increasing pressure in the section S1 of the interior of the container 20 into which the cell suspension has been injected, and reducing the pressure in the other section S2 etc. Examples

A. Methods for measuring the melting point, density, and film thickness of polymer films employable as a first polymer film configuring the first weld frame 10 and as a second polymer film configuring the second weld frame 12, together with methods for measuring the opening area ratio, opening diameter, and fiber diameter of filters employable as the filter 14 including the openings 14A, are as described below.

(A-1) Polymer Film Melting Point (Tm) Measurement Method.

Polymer film melting point measurement was performed by DSC (using a Q20 manufactured by TA instruments). Conditions of DSC measurement are in a nitrogen atmosphere (50 ml/min); over a measurement temperature range of from 30°C to 200°C; and a speed of temperature rise of 10°C/min. The temperature at the peak position of the endothermic peak indicating melting in DSC was employed as the melting point of the polymer film.

(A-2) Polymer Film Density Measurement Method

The measurement of the density of the polymer film was performed using a buoyancy weighing method. The buoyancy weighing method used scales (Blance XS105 manufactured by Mettler Toledo) with attached specific weight measurement jig (manufactured by Mettler Toledo) to weigh the polymer film in air, and then to weigh the polymer film in ethanol. The liquid temperature was measured and then the density of ethanol found using the method described in publication (Dweight E. Gray, American Institute of Physics Handbook, McGraw-Hill Book Company Inc., 1957), and then the density of the polymer film calculated by the following equation.

$$\rho = \{A/(A-B)\} \times (\rho 0 - d) + d$$

In the equation, $\rho$ is the density of the sample, A is the weight in air, B is the weight in a liquid, $\rho 0$ is the density of the liquid, and d is the density of air ($0.002$ g/cm$^3$).

(A-3) Polymer Film Film-Thickness Measurement Method

For the film thicknesses of the polymer films, values in a catalogue provided by a film maker were employed.

(A-4) Filter Opening Area Ratio Measurement Method

For the opening area ratio of the filter, values in the catalogue provided by the filter maker were employed.

(A-5) Filter Opening Diameter Measurement Method

For the diameter of the openings in the filter, values in the catalogue provided by the filter maker were employed.

(A-6) Filter Fiber Diameter Measurement Method

For the fiber diameter of the filter, values in the catalogue provided by the filter maker were employed.

B. Filtration Filter 16 Production Method

In a state in which the external periphery of the filter 14 was sandwiched around the entire periphery between the first polymer film configuring the first weld frame 10 and the second polymer film configuring the second weld frame 12, an impulse sealer (SURE NL-102JW manufactured by Ishizaki Electric Manufacturing Co., Ltd.) was employed for heating so as to weld the first weld frame 10, the filter 14, and the second weld frame 12 together through the weld portion 34. The impulse sealer was set to 230°C, this being not higher than the melting point of the filter 14 made from PET and having a melting point at least that of a polyethylene film. The weld portion 34 was formed around the entire periphery of the portion of the filter 14 sandwiched between the first weld frame 10 and the second weld frame 12. The filtration filter 16 produced in this manner includes a portion of filter that exhibits the function of a filter at the portion not sandwiched between the first weld frame 10 and the second weld frame 12, namely, at a portion corresponding to the first through hole 10A and the second through hole 12A. Such filtration filters 16 were produced while changing the various substances of the first polymer film configuring the first weld frame 10, of the second polymer film configuring the second weld frame 12, and of the filter 14, so as to produce filters of the substances listed in the following Examples 1 to 18, and Comparative Examples 1 to 18. Note that Comparative Examples 19 to 20 are examples in which the first polymer film and the second polymer film configuring the second weld frame 12 have been welded together, and, albeit not being equivalent to the filtration filter 16, the weld strengths thereof were evaluated similarly.

C. Weld Strength Measurement Method

The weld strength of the weld portion 34 in the filtration filter 16 was measured using an evaluation model described in detail below.

Measurement of weld strength was executed by a peeling test according to JIS Z 0238. The weld portion 34 of the filtration filter 16 welded by impulse sealer was cut into three pieces of length 25 mm and width 15 mm. A 180° peeling test was then performed thereon using a tensile tester (Ez-Test-Ez-SX manufactured by Shimadazu Corporation). The tensile tester was set with a pulling speed of 10 mm/s and a chuck-to-chuck distance of 20 mm/s. The weld strength (N/15 mm) was found by the maximum load before peeling or breaking of the weld portion 34 occurred, and was evaluated as the average value for samples (N=3).

Note that a standard value of JIS Z 0238 is 23 N/15 mm, and the filtration filter 16 of the technology disclosed herein also employed this numerical value as a standard to the weld strength that should be achieved for the weld portion 34.

Example 1

A filter 14 (No. T-380T manufactured by NBC Meshtec Incorporated: opening diameter 28 $\mu$m, fiber diameter 35 $\mu$m, opening area ratio 23%, Tm = 254°C, length 80 mm, width 15 mm) made from polyethylene terephthalate (PET) was sandwiched between two sheets of high density polyethylene film (HDPE, HD manufactured by Tamapoly Co., Ltd.: Tm = 131°C, density 0.912 g/cm$^3$, film thicknesses 200 $\mu$m, length 80 mm, width 15 mm) and then welded in this sandwiched state to obtain a filtration filter 16. The weld strength of a weld portion 34 thereof was 32.6 N/15 mm.

Example 2

A filter 14 (No. T-380T manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two sheets of high density polyethylene film (HDPE, POLYELITE EH manufactured by Hosokawa Yoko Co., Ltd.: Tm = 126°C, density 0.947 g/cm$^3$, film thicknesses 200 $\mu$m, length 80 mm, width 15 mm) and then welded in this sandwiched state to obtain a filtration filter 16. The weld strength of a weld portion 34 thereof was 33.3 N/15 mm.

Example 3

Two sheets of linear low density polyethylene film (LLDPE, HC#100 manufactured by Mitsui Chemicals Tohcello Incorporated: Tm = 124°C, density 0.922 g/cm$^3$, film thickness 100 $\mu$m, length 80 mm, width 15 mm) were superimposed and welded together to prepare a film having a film thickness of 200 $\mu$m. A filter 14 (No. T-380T manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two of these sheets of linear low density polyethylene films of film thickness 200 $\mu$m and then welded in this sandwiched state to obtain a filtration filter 16. The weld strength of a weld portion 34 thereof was 30.5 N/15 mm.

Example 4

Two sheets of linear low density polyethylene film (LLDPE, L4102 manufactured by Toyobo Co., Ltd.: Tm = 123°C, density 0.907 g/cm$^3$, film thickness 100 $\mu$m, and L4102, length 80 mm, width 15 mm) were superimposed and welded together to prepare a film having a film thickness of 200 $\mu$m. A filter 14 (No. T-380T manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two of these sheets of linear low density polyethylene films of film thickness 200 $\mu$m and then welded in this sandwiched state to obtain a filtration filter 16. The weld strength of a weld portion 34 thereof was 33.7 N/15 mm.

Example 5

Two sheets of linear low density polyethylene film (LLDPE, SE620L manufactured by Tamapoly Co., Ltd.: Tm = 113°C, density 0.921 g/cm$^3$, film thicknesses 140 $\mu$m, and SE620L, length 80 mm, width 15 mm) were superimposed and welded together to prepare a film having a film thickness of 280 $\mu$m. A filter 14 (No. T-380T manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two of these sheets of linear low density polyethylene films of film thickness 280 $\mu$m and then welded in this sandwiched state to obtain a filtration filter 16. The weld strength of a weld portion 34 thereof was 26.7 N/15 mm.

Example 6

A filter 14 (No. T-380T manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two sheets of linear low density polyethylene (LLDPE, SE620L manufactured by Tamapoly Co., Ltd.: Tm = 113°C, density 0.921 g/cm$^3$, film thicknesses 140 $\mu$m, and SE620L, length 80 mm, width 15 mm) and then welded in this sandwiched state to obtain a filtration filter 16. The weld strength of a weld portion 34 thereof was 20.2 N/15 mm.

Comparative Example 1

A filter 14 (No. T-380T manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two sheets of low density polyethylene (LDPE, V-2 manufactured by Tamapoly Co., Ltd.: Tm = 112°C, density 0.923 g/cm$^3$, film thicknesses 200 $\mu$m, length 80 mm, width 15 mm) and then welded in this sandwiched state to obtain a filtration filter 16. The weld strength of a weld portion 34 thereof was 13.9 N/15 mm.

Comparative Example 2

A filter 14 (No. T-380T manufactured by NBC Meshtec Incorporated: opening diameter 28 $\mu$m, fiber diameter 35 $\mu$m, opening area ratio 23%, Tm = 254°C, length 28 mm, width 15 mm) made from PET was sandwiched between two sheets of low density polyethylene (LDPE, POLYELITE EL manufactured by Hosokawa Yoko Co., Ltd.: Tm = 115°C, density 0.907 g/cm$^3$, film thicknesses 250 $\mu$m, length 80 mm, width 15 mm) and then welded in this sandwiched state to obtain a filtration filter 16. The weld strength of a weld portion 34 thereof was 13.9 N/15 mm.

Comparative Example 3

A filter 14 (No. T-380T manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two sheets of low density polyethylene (LDPE, manufactured by Sanplatec Co., Ltd.: Tm = 110°C, density 0.915 g/cm$^3$, film thicknesses 300 $\mu$m, length 80 mm, width 15 mm) and then welded in this sandwiched state to obtain a filtration filter 16. The weld strength of a weld portion 34 thereof was 14.9 N/15 mm.

Comparative Example 4

A filter 14 (No. T-380T manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two sheets of low density polyethylene (LDPE, manufactured by Sanplatec Co., Ltd.: Tm = 111°C, density 0.915 g/cm$^3$, film thicknesses 500 $\mu$m, length 80 mm, width 15 mm) and then welded in this sandwiched state to obtain a filtration filter 16. The weld strength of a weld portion 34 thereof was 14.4 N/15 mm.

Comparative Example 5

A filter 14 (No. T-380T manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two sheets of linear low density polyethylene (LLDPE, HC#100 manufactured by Mitsui Chemicals Tohcello Incorporated: Tm = 124°C, density 0.922 g/cm$^3$, film thickness 100 $\mu$m, length 80 mm, width 15 mm) and then welded in this sandwiched state to obtain a filtration filter 16. The weld strength of a weld portion 34 thereof was 12.3 N/15 mm.

Comparative Example 6

A filter 14 (No. T-380T manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two sheets of linear low density polyethylene film (LLDPE, L4102 manufactured by Toyobo Co., Ltd.: Tm = 123°C, density 0.907 g/cm$^3$, film thickness 100 $\mu$m, length 80 mm, width 15 mm) and then welded in this sandwiched state to obtain a filtration filter 16. The weld strength of a weld portion 34 thereof was 16.0 N/15 mm.

The results from Examples 1 to 6 and from Comparative Examples 1 to 6 are listed as Table 1.

Table 1

| Example or Comparative Example | Filter | | | | | | Film | | | | | Weld strength (N/15m m) | Comment |
|---|---|---|---|---|---|---|---|---|---|---|---|---|---|
| | Material | Maker | Product No. | Opening Diameter ($\mu$m) | Fiber Diameter ($\mu$m) | Mesh Opening (%) | Material | Maker | Product No. | Film Thick. ($\mu$m) | Tm (°C) | | |
| Example 1 | PET | NBC Meshtec Inc. | T-380T | 28 | 35 | 23 | HDPE | Tamapoly | HD | 200 | 131 | 32.6 | Employed sample: two sheets superimposed and welded together |
| Example 2 | | | | | | | | Hosokawa Yoko | POLYELITE EH | 200 | 126 | 33.3 | |
| Example 3 | | | | | | | LLDPE | Mitsui Chemicals Tohcello | HC#100 | 200 | 124 | 30.5 | |
| Example 4 | | | | | | | | Toyobo | L4102 | 200 | 123 | 33.7 | |
| Example 5 | | | | | | | | Tamapoly | SE620L | 280 | 113 | 26.7 | |
| Example 6 | | | | | | | | Tamapoly | SE620L | 140 | 113 | 20.2 | |
| Comparative Example 1 | PET | NBC Meshtec Inc. | T-380T | 28 | 35 | 23 | LDPE | Tamapoly | V-2 | 200 | 112 | 13.9 | |
| Comparative Example 2 | | | | | | | | Hosokawa Yoko | POLYELITE EL | 250 | 115 | 13.9 | |
| Comparative Example 3 | | | | | | | | Sanplatec | - | 300 | 110 | 14.9 | |
| Comparative Example 4 | | | | | | | | Sanplatec | - | 500 | 111 | 14.4 | |
| Comparative Example 5 | PET | NBC Meshtec Inc. | T-380T | 28 | 35 | 23 | LLDPE | Mitsui Chemicals Tohcello | HC#100 | 100 | 124 | 12.3 | |
| Comparative Example 6 | | | | | | | | Toyobo | L4102 | 100 | 123 | 16.0 | |

Example 7

A filter 14 (03-30/18 manufactured by Sefar: opening diameter 30 $\mu$m, fiber diameter 40 $\mu$m, opening area ratio 18%, Tm = 252°C, length 80 mm, width 15 mm) made from NYLON was sandwiched between two sheets of high density polyethylene film (HDPE, HD manufactured by Tamapoly Co., Ltd.: Tm = 131°C, density 0.912 g/cm$^3$, film thicknesses 200 $\mu$m, length 80 mm, width 15 mm) and then welded in this sandwiched state to obtain a filtration filter 16. The weld strength of a weld portion 34 thereof was 29.0 N/15 mm.

Example 8

Two sheets of linear low density polyethylene (LLDPE, HC#100 manufactured by Mitsui Chemicals Tohcello Incorporated: Tm = 124°C, density 0.922 g/cm$^3$, film thickness 100 $\mu$m, length 80 mm, width 15 mm) were superimposed and welded together, and a film having a film thickness of 200 $\mu$m was produced thereby. A filter 14 made from NYLON (03-30/18 manufactured by Sefar: opening diameter 30 $\mu$m, fiber diameter 40 $\mu$m, opening area ratio 18%, Tm = 252°C, length 80 mm, width 15 mm) was then sandwiched between two of these sheets of the linear low density polyethylene film having a film thickness of 200 $\mu$m, and welded in this sandwiched state to obtain a filtration filter 16. The weld strength of a weld portion 34 thereof was 23.4 N/15 mm.

Comparative Example 7

A filter 14 (03-30/18 manufactured by Sefar: opening diameter 30 $\mu$m, fiber diameter 40 $\mu$m, opening area ratio 18%, Tm = 252°C, length 80 mm, width 15 mm) made from NYLON was sandwiched between two sheets of low density polyethylene film (LDPE, V-2 manufactured by Tamapoly Co., Ltd.: Tm = 112°C, density 0.923 g/cm$^3$, film thicknesses 200 $\mu$m, length 80 mm, width 15 mm) and then welded in this sandwiched state to obtain a filtration filter 16. The weld strength of a weld portion 34 thereof was 12.0 N/15 mm.
The results from Examples 7, 8 and Comparative Example 7 are listed in Table 2.

Table 2

| Example or Comparative Example | Filter | | | | | | Film | | | | | Weld strength (N/15mm) | Comment |
|---|---|---|---|---|---|---|---|---|---|---|---|---|---|
| | Material | Maker | Product No. | Opening Diameter ($\mu$m) | Fiber Diameter ($\mu$m) | Mesh Opening (%) | Material | Maker | Product No. | Film Thickness ($\mu$m) | Tm (°C) | | |
| Example 7 | | | | | | | HDPE | Tamapoly | HD | 200 | 131 | 29.0 | |
| Example 8 | NYLON | Sefar | 03-30/18 | 30 | 40 | 18 | LLDPE | Mitsui Chemicals Tohcello | HC#100 | 200 | 124 | 23.4 | Employed sample: two sheets superimposed and welded together |
| Comparative Example 7 | NYLON | Sefar | 03-30/18 | 30 | 40 | 18 | LDPE | Tamapoly | V-2 | 200 | 112 | 12 | |

Example 9

A filter 14 (PET 24, manufactured by Sefar: opening diameter 21 $\mu$m, fiber diameter 41 $\mu$m, opening area ratio 12%, Tm = 257°C, length 80 mm, width 15 mm) made from PET was sandwiched between two sheets of high density polyethylene film (HDPE, HD manufactured by Tamapoly Co., Ltd.: Tm = 131°C, density 0.912 g/cm$^3$, film thicknesses 200 $\mu$m, length 80 mm, width 15 mm) and then welded in this sandwiched state to obtain a filtration filter 16. The weld strength of a weld portion 34 thereof was 24.2 N/15 mm.

Example 10

Two sheets of linear low density polyethylene (LLDPE, HC#100 manufactured by Mitsui Chemicals Tohcello Incorporated: Tm = 124°C, density 0.922 g/cm$^3$, film thickness 100 $\mu$m, length 80 mm, width 15 mm) were superimposed and welded together, and a film having a film thickness of 200 $\mu$m was produced thereby. A filter 14 (PET 24 manufactured by Sefar: opening diameter 21 $\mu$m, fiber diameter 41 $\mu$m, opening area ratio 12%, Tm = 257°C, length 80 mm, width 15 mm) made from PET was sandwiched between two sheets of such linear low density polyethylene film having a film thickness of 200 $\mu$m and welded in this sandwiched state to obtain a filtration filter 16. The weld strength of a weld portion 34 thereof was 24.2 N/15 mm.

Example 11

A filter 14 (07-27/19, manufactured by Sefar: opening diameter 27 $\mu$m, fiber diameter 35 $\mu$m, opening area ratio 19%, Tm = 256°C, length 80 mm, width 15 mm) made from PET was sandwiched between two sheets of high density polyethylene film (HDPE, HD manufactured by Tamapoly Co., Ltd.: Tm = 131°C, density 0.912 g/cm$^3$, film thicknesses 200 $\mu$m, length 80 mm, width 15 mm) and then welded in this sandwiched state to obtain a filtration filter 16. The weld strength of a weld portion 34 thereof was 32.7 N/15 mm.

Example 12

Two sheets of linear low density polyethylene (LLDPE, HC#100 manufactured by Mitsui Chemicals Tohcello Incorporated: Tm = 124°C, density 0.922 g/cm$^3$, film thickness 100 $\mu$m, length 80 mm, width 15 mm) were superimposed and welded together, and a film having a film thickness of 200 $\mu$m was produced thereby. A filter 14 (07-27/19, manufactured by Sefar: opening diameter 27 $\mu$m, fiber diameter 35 $\mu$m, opening area ratio 19%, Tm = 256°C, length 80 mm, width 15 mm) made from PET was sandwiched between two of these sheets of linear low density polyethylene film having a film thickness of 200 $\mu$m and welded in this sandwiched state to obtain a filtration filter 16. The weld strength of a weld portion 34 thereof was 29.4 N/15 mm.

Example 13

A filter 14 (T-380T, manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two sheets of high density polyethylene film (HDPE, HD manufactured by Tamapoly Co., Ltd.: Tm = 131°C, density 0.912 g/cm$^3$, film thicknesses 200 $\mu$m, length 80 mm, width 15 mm) and then welded in this sandwiched state to obtain a filtration filter 16. The weld strength of a weld portion 34 thereof was 32.6 N/15 mm.

Example 14

Two sheets of linear low density polyethylene (LLDPE, HC#100 manufactured by Mitsui Chemicals Tohcello Incorporated: Tm = 124°C, density 0.922 g/cm$^3$, film thickness 100 $\mu$m, length 80 mm, width 15 mm) were superimposed and welded together, and a film having a film thickness of 200 $\mu$m was produced thereby. A filter 14 (T-380T, manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two of these sheets of linear low density polyethylene film having a film thickness of 200 $\mu$m and welded in this sandwiched state to obtain a filtration filter 16. The weld strength of a weld portion 34 thereof was 30.5 N/15 mm.

Example 15

A filter 14 (T-180T, manufactured by NBC Meshtec Incorporated: opening diameter 86 $\mu$m, fiber diameter 55 $\mu$m, opening area ratio 37%, Tm = 255°C, length 80 mm, width 15 mm) made from PET was sandwiched between two sheets of high density polyethylene film (HDPE, HD manufactured by Tamapoly Co., Ltd.: Tm = 131°C, density 0.912 g/cm$^3$, film thicknesses 200 $\mu$m, length 80 mm, width 15 mm) and then welded in this sandwiched state to obtain a filtration filter 16. The weld strength of a weld portion 34 thereof was 52.2 N/15 mm.

Example 16

Two sheets of linear low density polyethylene (LLDPE, HC#100 manufactured by Mitsui Chemicals Tohcello Incorporated: Tm = 124°C, density 0.922 g/cm$^3$, film thickness 100 $\mu$m, length 80 mm, width 15 mm) were superimposed and welded together, and a film having a film thickness of 200 $\mu$m was produced thereby. A filter 14 (T-180T, manufactured by NBC Meshtec Incorporated: opening diameter 86 $\mu$m, fiber diameter 55 $\mu$m, opening area ratio 37%, Tm = 255°C, length 80 mm, width 15 mm) made from PET was sandwiched between two of these sheets of linear low density polyethylene film having a film thickness of 200 $\mu$m and welded in this sandwiched state to obtain a filtration filter 16. The weld strength of a weld portion 34 thereof was 35.8 N/15 mm.

Example 17

A filter 14 (T-100T, manufactured by NBC Meshtec Incorporated: opening diameter 183 $\mu$m, fiber diameter 71 $\mu$m, opening area ratio 52%, Tm = 255°C, length 80 mm, width 15 mm) made from PET was sandwiched between two sheets of high density polyethylene film (HDPE, HD manufactured by Tamapoly Co., Ltd.: Tm = 131°C, density 0.912 g/cm$^3$, film thicknesses 200 $\mu$m, length 80 mm, width 15 mm) and then welded in this sandwiched state to obtain a filtration filter 16. The weld strength of a weld portion 34 thereof was 53.6 N/15 mm.

Example 18

Two sheets of linear low density polyethylene (LLDPE, HC#100 manufactured by Mitsui Chemicals Tohcello Incorporated: Tm = 124°C, density 0.922 g/cm$^3$, film thickness 100 $\mu$m, length 80 mm, width 15 mm) were superimposed and welded together, and a film having a film thickness of 200 $\mu$m was produced thereby. A filter 14 (T-100T, manufactured by NBC Meshtec Incorporated: opening diameter 183 $\mu$m, fiber diameter 71 $\mu$m, opening area ratio 52%, Tm = 255°C, length 80 mm, width 15 mm) made from PET was sandwiched between two sheets of such linear low density polyethylene film having a film thickness of 200 $\mu$m and welded in this sandwiched state to obtain a filtration filter 16. The weld strength of a weld portion 34 thereof was 35.6 N/15 mm.

Comparative Example 8

A filter 14 (PET 6-HD manufactured by Sefar: opening diameter 6 $\mu$m, fiber diameter 34 $\mu$m, opening area ratio 5%, Tm = 257°C, length 80 mm, width 15 mm) made from PET was sandwiched between two sheets of high density polyethylene film (HDPE, HD manufactured by Tamapoly Co., Ltd.: Tm = 131°C, density 0.912 g/cm$^3$, film thicknesses 200 $\mu$m, length 80 mm, width 15 mm) and then welded in this sandwiched state to obtain a filtration filter 16. The weld strength of a weld portion 34 thereof was 13.1 N/15 mm.

Comparative Example 9

Two sheets of linear low density polyethylene (LLDPE, HC#100 manufactured by Mitsui Chemicals Tohcello Incorporated: Tm = 124°C, density 0.922 g/cm$^3$, film thickness 100 $\mu$m, length 80 mm, width 15 mm) were superimposed and welded together, and a film having a film thickness of 200 $\mu$m was produced thereby. A filter 14 (PET 6-HD manufactured by Sefar: opening diameter 6 $\mu$m, fiber diameter 34 $\mu$m, opening area ratio 5%, Tm = 257°C, length 80 mm, width 15 mm) made from PET was sandwiched between two sheets of such linear low density polyethylene film having a film thickness of 200 $\mu$m and welded in this sandwiched state to obtain a filtration filter 16. The weld strength of a weld portion 34 thereof was 12.3 N/15 mm.

Comparative Example 10

A filter 14 (PET 6-HD manufactured by Sefar: opening diameter 6 $\mu$m, fiber diameter 34 $\mu$m, opening area ratio 5%, Tm = 257°C, length 80 mm, width 15 mm) made from PET was sandwiched between two sheets of low density polyethylene film (LDPE, V-2 manufactured by Tamapoly Co., Ltd.: Tm = 112°C, density 0.923 g/cm$^3$, film thicknesses 200 $\mu$m, length 80 mm, width 15 mm) and then welded in this sandwiched state to obtain a filtration filter 16. The weld strength of a weld portion 34 thereof was 6.0 N/15 mm.

Comparative Example 11

A filter 14 (PET 15 manufactured by Sefar: opening diameter 15 $\mu$m, fiber diameter 37 $\mu$m, opening area ratio 9%, Tm = 257°C, length 80 mm, width 15 mm) made from PET was sandwiched between two sheets of high density polyethylene film (HDPE, HD manufactured by Tamapoly Co., Ltd.: Tm = 131°C, density 0.912 g/cm$^3$, film thicknesses 200 $\mu$m, length 80 mm, width 15 mm) and then welded in this sandwiched state to obtain a filtration filter 16. The weld strength of a weld portion 34 thereof was 18.5 N/15 mm.

Comparative Example 12

Two sheets of linear low density polyethylene (LLDPE, HC#100 manufactured by Mitsui Chemicals Tohcello Incorporated: Tm = 124°C, density 0.922 g/cm$^3$, film thickness 100 $\mu$m, length 80 mm, width 15 mm) were superimposed and welded together, and a film having a film thickness of 200 $\mu$m was produced thereby. A filter 14 (PET 15 manufactured by Sefar: opening diameter 15 $\mu$m, fiber diameter 37 $\mu$m, opening area ratio 9%, Tm = 257°C, length 80 mm, width 15 mm) made from PET was sandwiched between two sheets of such a linear low density polyethylene film having a film thickness of 200 $\mu$m and welded in this sandwiched state to obtain a filtration filter 16. The weld strength of a weld portion 34 thereof was 17.1 N/15 mm.

Comparative Example 13

A filter 14 (PET 15 manufactured by Sefar: opening diameter 15 $\mu$m, fiber diameter 37 $\mu$m, opening area ratio 9%, Tm = 257°C, length 80 mm, width 15 mm) made from PET was sandwiched between two sheets of low density polyethylene (LDPE, V-2 manufactured by Tamapoly Co., Ltd.: Tm = 112°C, density 0.923 g/cm$^3$, film thicknesses 200 $\mu$m, length 80 mm, width 15 mm) and then welded in this sandwiched state to obtain a filtration filter 16. The weld strength of a weld portion 34 thereof was 8.1 N/15 mm.

Comparative Example 14

A filter 14 (PET 24 manufactured by Sefar: opening diameter 21 $\mu$m, fiber diameter 41 $\mu$m, opening area ratio 12%, Tm = 257°C, length 80 mm, width 15 mm) made from PET was sandwiched between two sheets of low density polyethylene film (LDPE, V-2 manufactured by Tamapoly Co., Ltd.: Tm = 112°C, density 0.923 g/cm$^3$, film thicknesses 200 $\mu$m, length 80 mm, width 15 mm) and then welded in this sandwiched state to obtain a filtration filter 16. The weld strength of a weld portion 34 thereof was 11.1 N/15 mm.

Comparative Example 15

A filter 14 (07-27/19 manufactured by Sefar: opening diameter 27 $\mu$m, fiber diameter 35 $\mu$m, opening area ratio 19%, Tm = 256°C, length 80 mm, width 15 mm) made from PET was sandwiched between two sheets of low density polyethylene film (LDPE, V-2 manufactured by Tamapoly Co., Ltd.: Tm = 112°C, density 0.923 g/cm$^3$, film thicknesses 200 $\mu$m, length 80 mm, width 15 mm) and then welded in this sandwiched state to obtain a filtration filter 16. The weld strength of a weld portion 34 thereof was 13.8 N/15 mm.

Comparative Example 16

A filter 14 (T-380T, manufactured by NBC Meshtec Incorporated: opening diameter 28 $\mu$m, fiber diameter 35 $\mu$m, opening area ratio 23%, Tm = 254°C, length 80 mm, width 15 mm) made from PET was sandwiched between two sheets of low density polyethylene film (LDPE, V-2 manufactured by Tamapoly Co., Ltd.: Tm = 112°C, density 0.923 g/cm$^3$, film thicknesses 200 $\mu$m, length 80 mm, width 15 mm) and then welded in this sandwiched state to obtain a filtration filter 16. The weld strength of a weld portion 34 thereof was 13.9 N/15 mm.

Comparative Example 17

A filter 14 (T-180T, manufactured by NBC Meshtec Incorporated: opening diameter 86 $\mu$m, fiber diameter 55 $\mu$m, opening area ratio 37%, Tm = 255°C, length 80 mm, width 15 mm) made from PET was sandwiched between two sheets of low density polyethylene film (LDPE, V-2 manufactured by Tamapoly Co., Ltd.: Tm = 112°C, density 0.923 g/cm$^3$, film thicknesses 200 $\mu$m, length 80 mm, width 15 mm) and then welded in this sandwiched state to obtain a filtration filter 16. The weld strength of a weld portion 34 thereof was 21.6 N/15 mm.

Comparative Example 18

A filter 14 (T-100T, manufactured by NBC Meshtec Incorporated: opening diameter 183 $\mu$m, fiber diameter 71 $\mu$m, opening area ratio 52%, Tm = 255°C, length 80 mm, width 15 mm) made from PET was sandwiched between two sheets of low density polyethylene film (LDPE, V-2 manufactured by Tamapoly Co., Ltd.: Tm = 112°C, density 0.923 g/cm$^3$, film thicknesses 200 $\mu$m, length 80 mm, width 15 mm) and then welded in this sandwiched state to obtain a filtration filter 16. The weld strength of a weld portion 34 thereof was 32.1 N/15 mm.

Results of Examples 9 to 18 and Comparative Examples 8 to 18 are listed in Table 3.

Table 3

| Example or Comparative Example | Filter | | | | | | Film | | | | | Weld strength (N/15mm) | Comment |
|---|---|---|---|---|---|---|---|---|---|---|---|---|---|
| | Material | Maker | Product No. | Opening Diameter ($\mu$m) | Fiber Diameter ($\mu$m) | Mesh Opening (%) | Material | Maker | Product No. | Film Thick. ($\mu$m) | Tm (°C) | | |
| Example 9 | PET | Sefar | PET24 | 21 | 41 | 12 | HDPE | Tamapoly | HD | 200 | 131 | 24.2 | When LLDPE was employed the employed sample was two sheets superimposed and welded together |
| Example 10 | | | | | | | LLDPE | Mitsui Chemicals Tohcello | HC#100 | 200 | 124 | 24.2 | |
| Example 11 | PET | Sefar | 07-27/19 | 27 | 35 | 19 | HDPE | Tamapoly | HD | 200 | 131 | 32.7 | |
| Example 12 | | | | | | | LLDPE | Mitsui Chemicals Tohcello | HC#100 | 200 | 124 | 29.4 | |
| Example 13 | PET | NBC Meshtec Inc. | T-380T | 28 | 35 | 23 | HDPE | Tamapoly | HD | 200 | 131 | 32.6 | |
| Example 14 | | | | | | | LLDPE | Mitsui Chemicals Tohcello | HC#100 | 200 | 124 | 30.5 | |
| Example 15 | PET | NBC Meshtec Inc. | T-180T | 86 | 55 | 37 | HDPE | Tamapoly | HD | 200 | 131 | 52.2 | |
| Example 16 | | | | | | | LLDPE | Mitsui Chemicals Tohcello | HC#100 | 200 | 124 | 35.8 | |
| Example 17 | PET | NBC Meshtec Inc. | T-100T | 183 | 71 | 52 | HDPE | Tamapoly | HD | 200 | 131 | 53.6 | |
| Example 18 | | | | | | | LLDPE | Mitsui Chemicals Tohcello | HC#100 | 200 | 124 | 35.6 | |

| Example or Comparative Example | Filter | | | | | | Film | | | | | Weld strength (N/15mm) | Comment |
|---|---|---|---|---|---|---|---|---|---|---|---|---|---|
| | Material | Maker | Product No. | Opening Di-ameter ($\mu$m) | Fiber Diam-eter ($\mu$m) | Mesh Opening (%) | Material | Maker | Product No. | Film Thick. ($\mu$m) | Tm (°C) | | |
| Comparative Example 8 | PET | Sefar | PET6-HD | 6 | 34 | 5 | HDPE | Tamapoly | HD | 200 | 131 | 13.1 | |
| Comparative Example 9 | | | | | | | LLDPE | Mitsui Chemicals Tohcello | HC#100 | 200 | 124 | 12.3 | |
| Comparative Example 10 | | | | | | | LDPE | Tamapoly | V-2 | 200 | 112 | 6.0 | |
| Comparative Example 11 | PET | Sefar | PET15 | 15 | 37 | 9 | HDPE | Tamapoly | HD | 200 | 131 | 18.5 | When LLDPE was employed the employed sample was two sheets superimposed and welded together |
| Comparative Example 12 | | | | | | | LLDPE | Mitsui Chemicals Tohcello | HC#100 | 200 | 124 | 17.1 | |
| Comparative Example 13 | | | | | | | LDPE | Tamapoly | V-2 | 200 | 112 | 8.1 | |
| Comparative Example 14 | PET | Sefar | PET24 | 21 | 41 | 12 | LDPE | Tamapoly | V-2 | 200 | 112 | 11.1 | |
| Comparative Example 15 | PET | Sefar | 07-27/19 | 27 | 35 | 19 | LDPE | Tamapoly | V-2 | 200 | 112 | 13.8 | |
| Comparative Example 16 | PET | NBC Meshtec Inc. | T-380T | 28 | 35 | 23 | LDPE | Tamapoly | V-2 | 200 | 112 | 13.9 | |
| Comparative Example 17 | PET | NBC Meshtec Inc. | T-180T | 86 | 55 | 37 | LDPE | Tamapoly | V-2 | 200 | 112 | 21.6 | |
| Comparative Example 18 | PET | NBC Meshtec Inc. | T-100T | 183 | 71 | 52 | LDPE | Tamapoly | V-2 | 200 | 112 | 32.1 | |

Sample were prepared by superimposing and welding together two sheets of film without sandwiching a filter 14 and measurements performed thereon to confirm weld strengths for cases in which hypothetical filters having an opening area ratio of 100% are sandwiched.

Comparative Example 19

Two sheets of high density polyethylene film (HDPE, HD manufactured by Tamapoly Co., Ltd.: Tm = 131°C, density 0.912 g/cm$^3$, film thicknesses 200 $\mu$m, length 80 mm, width 15 mm) were superimposed and then opposing faces welded together before measuring the weld strength thereof. The weld strength was 51.8 N/15 mm.

Comparative Example 20

Two sheets of linear low density polyethylene film (LLDPE, HC#100 manufactured by Mitsui Chemicals Tohcello Incorporated: Tm = 124°C, density 0.922 g/cm$^3$, film thickness 100 $\mu$m, length 80 mm, width 15 mm) were superimposed and welded together to prepare a film having a film thickness of 200 $\mu$m. Two sheets of such linear low density polyethylene film having a film thickness of 200 $\mu$m were superimposed and then opposing faces welded together before measuring the weld strength thereof. The weld strength was 33.5 N/15 mm.

Comparative Example 21

Two sheets of low density polyethylene film (LDPE, V-2 manufactured by Tamapoly Co., Ltd.: Tm = 112°C, density 0.923 g/cm$^3$, film thicknesses 200 $\mu$m, length 80 mm, width 15 mm) were superimposed and then opposing faces welded together before measuring the weld strength thereof. The weld strength was 27.9 N/15 mm.

The results of Comparative Examples 19 to 21 are listed in Table 4.

Table 4

| Comparative Example | PE Film 1 | | | | | | PE Film 2 | | | | | Weld Strength of PE Film 1 and PE Film 2 (N/15 mm) | Comment |
|---|---|---|---|---|---|---|---|---|---|---|---|---|---|
| | Material | Maker | Product No. | Opening Diameter ($\mu$m) | Fiber Diameter ($\mu$m) | Mesh Opening (%) | Material | Maker | Product No. | Film Thick. ($\mu$m) | Tm (°C) | | |
| Comparative Example 19 | HDPE | Tamapoly | HD | 200 | 131 | 100 | HDPE | Tamapoly | HD | 200 | 131 | 51.8 | |
| Comparative Example 20 | LLDPE | Mitsui Chemicals Tohcello | HC#100 | 200 | 124 | 100 | LLDPE | Mitsui Chemicals Tohcello | HC#100 | 200 | 124 | 33.5 | Employed sample was two sheets superimposed and welded together |
| Comparative Example 21 | LDPE | Tamapoly | V-2 | 200 | 112 | 100 | LDPE | Tamapoly | V-2 | 200 | 112 | 27.9 | |

## EP 3 871 748 A1

Interpretation

Regarding the relationship between the material of the films and the weld strength to the PET filter 14, as is apparent from Table 1, the highest weld strength is achieved by employing HDPE as the material of the film, the lowest when LDPE is employed, and the weld strength is somewhere in between when LLDPE is employed. This is similarly apparent from the results of Table 2 in which NYLON is employed as the material of the filter 14.

Regarding the effect of the film thickness of the films to the weld strength between the films and the filter 14, although the film thickness of the films has substantially no effect in cases in which LDPE is employed as the material of the film, as in Comparative Examples 1 to 4 of Table 1. In contrast thereto, it is apparent that the weld strength is increased by a film thickness of the films of 200 $\mu$m compared to 100 $\mu$m in cases in which LLDPE is employed as the material of the films.

Such changes to the dependency on film thickness to the weld strength due to the substance of the filter 14 are not understood in the mechanism of the phenomenon of welding utilized in technology disclosed herein, and demonstrate that the technology disclosed herein relates to a technical field having low predictability.

It is apparent from Table 3 that the opening area ratio of the filter 14 affects the weld strength. Namely, at an opening area ratio of the filter 14 in a range of around from 10% to 40%, there is a tendency for higher opening area ratio to lead to a higher strength of weld to the film. However, in cases in which the material of the film is HDPE or LLDPE, it was confirmed that the weld strength tends to become saturated at an opening area ratio of about 40%. Functioning as a filter is no longer exhibited when the opening area ratio of the filter 14 exceeds 80%.

Example 19

D. Filter-Equipped Container Production

A filter 14 (07-27/19 manufactured by Sefar: opening diameter 27 $\mu$m, opening area ratio 19%, Tm = 256°C) made from PET was sandwiched between frames (size: external frame 300 mm × 200 mm, internal frame 270 mm × 170 mm, frame width at each side 15 mm) made from a high density polyethylene film (HDPE, POLYELITE EH manufactured by Hosokawa Yoko Co., Ltd.: Tm = 126°C, density 0.947 g/cm$^3$, film thicknesses 200 $\mu$m), and welding was performed at peripheral portions at 230°C using an impulse sealer. The filtration filter 16 including the PET filter produced in this manner was then, as illustrated in Fig. 4, welded along three sides to inside faces of a container 20 made from high density polyethylene sheets, so as to fix the filtration filter 16 including the PET filter 14 between two sheets of high density polyethylene. Then after attaching an injection port 30 and a discharge port 32 made from polyethylene to the high density polyethylene sheets by welding, a tube 36 made from polyvinyl chloride was attached to each port. Finally, a filter-equipped container 22 was produced in which the filtration filter 16 configured by the PET filter 14 had been welded to the interior of the container 20 by welding peripheral edge portions of the high density polyethylene sheets together at portions other than the portions sandwiching the filtration filter 16 configured by the PET filter 14.

Compressed air at 0.01 MPa was introduced from the polyvinylchloride tube 36 of the thus produced filter-equipped container 22 so as to pass through the injection port 30. When this was being performed the discharge port 32 on the discharge side was open, and a bag internal pressure was adjusted so as to be 0.01 MPa. A check was performed for air leaks from the welded portions by placing the inflated filter-equipped container 22 in water, and no air leaks from the filter-equipped container 22 were observed.

Operation and Advantageous Effects of Present Exemplary Embodiment

Explanation next follows regarding operation and advantageous effects of the present exemplary embodiment.

As described above, the filtration filter 16 is configured from the first weld frame 10, the second weld frame 12 disposed so as to oppose the first weld frame 10 in the thickness direction, and the filter 14 having and outer peripheral portion welded in a state sandwiched between the entire periphery of the first weld frame 10 and the entire periphery of the second weld frame 12. The first weld frame 10 is thus formed in a frame shape from a flexible film of polymer having a film thickness of at least 120 $\mu$m with the first through hole 10A penetrating through inside the frame in the thickness direction, and the second weld frame 12 is thus formed in a frame shape from a flexible film of polymer having a film thickness of at least 120 $\mu$m with the second through hole 12A penetrating through inside the frame in the thickness direction. Adopting a configuration in which the filter 14 is configured by a substance having a higher melting point than the first weld frame 10 and the second weld frame 12, and having an opening area ratio of from 10% to 80% arising from provision of the openings therein results in the first weld frame 10 and the second weld frame 12 being melted through from the two faces of the filter 14 so as to be connected together with the openings 14A penetrating through the filter 14 where there is no melted first weld frame 10 and the second weld frame 12. This accordingly enables the filter 14 to be strongly fixed with the opposing first weld frame 10 and second weld frame 12 joined together. The filter 14 is accordingly welded to the first weld frame 10 and the second weld frame 12 with a high weld strength, the weld portion 34 is not easily separated, and there is little concern that fluid might leak through the weld portion 34.

Moreover, the weld portion 34 is less liable to separate due to the first weld frame 10 and the second weld frame 12 being made from a polymer that includes a high density polyethylene having a melting point of from 120°C to 140°C, that includes a linear low density polyethylene having a melting point of from 105°C to 125°C, or that includes a mixture of both.

Furthermore, the weld portion 34 is even less liable to separate due to the first weld frame 10 and the second weld frame 12 being made from a polymer that is a high density polyethylene having a melting point of from 120°C to 140°C, that is a linear low density polyethylene having a melting point of from 105°C to 125°C, or that is a mixture of both.

Furthermore, fluid can be assured to flow through the filter 14 by welding the first weld frame 10, the filter 14, and the second weld frame 12 together in a state in which fluid in the filtration target is able to move in sequence through the first through hole 10A, the portions of the filter 14 not contacting either the first weld frame 10 or the second weld frame 12, and the second through hole 12A.

Moreover, strength can be assured in the filter 14 itself due to configuring the filter 14 including at least one material from out of polyester, polyamide, polyolefin, polyether ether ketone, polyether sulfone, carbon fiber, or metal.

Furthermore, the filter 14 has flexibility due to the filter 14 being either a woven product or a knitted product. In the filter-equipped container 22 the filtration filter 16 is welded so as to section the interior of the container 20 made from polymer. This facilitates the filter-equipped container 22 giving and crumpling, facilitating removal of foreign matter from the cell suspension.

Furthermore, making the opening diameter of the openings 14A in the filter 14 from 10 $\mu$m to 200 $\mu$m enables residue and the target cells to be trapped while preventing the filter 14 from becoming blocked.

Moreover, the filter 14 is formed in a rectangular sheet shape and so is easily processed when producing the filter 14.

Furthermore, making the shape of the first weld frame 10 and the shape of the second weld frame 12 substantially identical enables the productivity to be raised when producing the filtration filter 16.

Furthermore, the container 20 is configured by opposing two polymer sheets of substantially the same rectangular shape and welding the peripheral edge portions of the polymer sheets together. The first weld frame 10 of the filtration filter 16 provided at the interior of the container 20 is welded with a substantially U-shaped first weld portion 24 that is open in one direction when one of the polymer sheets of the container 20 is viewed in the thickness direction, and the second weld frame 12 of the filtration filter 16 is welded with a substantially U-shaped second weld portion 26 that is open towards another direction when the other polymer sheet of the container 20 is viewed in the thickness direction. The opening of the first weld portion 24 and the opening of the second weld portion 26 accordingly face in opposite directions. The fluid that has flowed into the filter 14 from the opening in the first weld portion 24 accordingly flows out through the opening of the second weld portion 26 without a large change in the direction of flow. Namely, flow of the fluid is facilitated so as to enable trapping of residue and the target cells to be performed with good efficiency.

Moreover, providing at least one of the injection port 30 or the discharge port 32 to the container 20 enables easy filling of fluid into the interior of the container 20. Providing the injection port 30 to one segment of the interior of the container 20 sectioned by the filtration filter 16 and providing the discharge port 32 to the other segment thereof enables cell suspension to be easily injected into the one section S1 of the interior of the container 20, and enables the cell suspension from which the foreign matter has been removed to be easily recovered from the other section S2. This moreover facilitates the cell suspension to be injected into the one section S1 of the interior of the container 20 in the filter-equipped container 22, cells to be filtered by the filtration filter 16, liquid for re-suspending cells to be injected into the one section S1 and cells to be re-suspended, and the cell suspension to be recovered from the one section S1.

Furthermore, due to the first weld frame 10 and the second weld frame 12 being welded at a temperature equal to or higher than the respective melting point of the first weld frame 10 or of the second weld frame 12 and equal to or lower than the melting point of the filter 14 while in a state in which the filter 14 is sandwiched between the entire periphery of the first weld frame 10 and the entire periphery of the second weld frame 12, the melted polymer of the first weld frame 10 and the second weld frame 12 can be caused to intrude into the openings 14A of the unmelted filter 14. The polymer can then be caused to solidify through heat dissipation, for example. The polymer of the first weld frame 10 and the second weld frame 12 that has intrude into the openings 14A accordingly acts as a so-called anchor, enabling an increase to be achieved in the weld strength of the filter 14 to the first weld frame 10 and the second weld frame 12.

Note that although in the filter-equipped container 22 described above a configuration is adopted in which the filter 14 is sandwiched between the first weld frame 10 and the second weld frame 12, there is no limitation thereto. Although not illustrated, a configuration may be adopted in which the filter 14 is directly welded to the interior of the container 20 so as to section the container 20, such that the interior of the container 20 is partitioned by the filter 14 in a manner in which pathway for fluid to move from a space configured between one face of the filter 14 and the interior of the container 20 to a space configured between the other face of the filter 14 and the interior of the container 20 is not present anywhere other than through the filter 14. Adopting such a configuration enables the function of a filtration unit to be realized while reducing the number of configuration components.

Industrial Applicability

The filter-equipped container 22 incorporating the filtration filter 16 of technology disclosed herein inside the container 20 may be employed, for example, to remove foreign matter from a cell suspension and to wash cells. The filtration filter 16 of technology disclosed herein and the filter-equipped container 22 employing the same are accordingly utilizable in manufacturing of filtration equipment, for example.

The entire content of the disclosure of Japanese Patent Application No. 2018-199182 filed on October 23, 2018, is incorporated by reference in the present specification. All publications, patent applications and technical standards mentioned in the present specification are incorporated by reference in the present specification to the same extent as if each individual publication, patent application, or technical standard was specifically and individually indicated to be incorporated by reference.

**Claims**

1.    A filtration filter, comprising:

a first weld frame configured by a flexible film that includes a polymer and that has a film thickness of at least 120 $\mu$m, the first weld frame being formed into a frame shape including a first through hole penetrating an inside of the frame shape in a thickness direction;

a second weld frame opposing the first weld frame in the thickness direction and being configured by a flexible film that includes a polymer and that has a film thickness of at least 120 $\mu$m, the second weld frame being formed into a frame shape including a second through hole penetrating an inside of the frame shape in the thickness direction at a position corresponding to the first through hole; and

a filter configured by a substance having a higher melting point than the first weld frame and the second weld frame, the filter being provided with openings and having an opening area ratio due to the openings of from 10% to 80%, and being welded to both the first weld frame and the second weld frame in a state in which an outer peripheral portion of the filter is sandwiched between an entire periphery of the first weld frame and an entire periphery of the second weld frame,

the first weld frame being configured by a polymer that includes either a high density polyethylene having a melting point of from 120°C to 140°C, a linear low density polyethylene having a melting point of from 105°C to 125°C, or a mixture including at least one of the high density polyethylene or the linear low density polyethylene.

2.    The filtration filter of claim 1, wherein the first weld frame is configured by a polymer that consists of either a high density polyethylene having a melting point of from 120°C to 140°C, a linear low density polyethylene having a melting point of from 105°C to 125°C, or a mixture including at least one of the high density polyethylene or the linear low density polyethylene.

3.    The filtration filter of claim 1 or claim 2, wherein the first weld frame, the filter, and the second weld frame are welded together in a state in which fluid to be filtrated is able to move through the first through hole, a portion of the filter not contacting either the first weld frame or the second weld frame, and the second through hole in this order.

4.    The filtration filter of any one of claim 1 to claim 3, wherein the second weld frame is configured by a polymer that includes either a high density polyethylene having a melting point of from 120°C to 140°C, a linear low density polyethylene having a melting point of from 105°C to 125°C, or a mixture including at least one of the high density polyethylene or the linear low density polyethylene.

5.    The filtration filter of any one of claim 1 to claim 3, wherein the second weld frame is configured by a polymer that consists of either a high density polyethylene having a melting point of from 120°C to 140°C, a linear low density polyethylene having a melting point of from 105°C to 125°C, or a mixture at least one of the high density polyethylene or the linear low density polyethylene.

6.    The filtration filter of any one of claim 1 to claim 5, wherein the filter includes at least one material selected from the group consisting of a polyester, a polyamide, a polyolefin, a polyether ether ketone, a polyether sulfone, a carbon fiber, and a metal.

7. The filtration filter of any one of claim 1 to claim 6, wherein the filter is a woven product or a knitted product.

8. The filtration filter of any one of claim 1 to claim 7, wherein a diameter of the openings in the filter is from 5 μm to 200 μm.

9. The filtration filter of any one of claim 1 to claim 8, wherein the filter has a rectangular sheet shape.

10. The filtration filter of any one of claim 1 to claim 9, wherein a shape of the first weld frame and a shape of the second weld frame are substantially identical.

11. The filtration filter of any one of claim 1 to claim 10, wherein the filter is welded to the first weld frame and the second weld frame in a state that the filter has been sandwiched between the entire periphery of the first weld frame and the entire periphery of the second weld frame by being heated to a temperature equal to or higher than the melting point of the first weld frame or the second weld frame and equal to or lower than the melting point of the filter.

12. A filter-equipped container comprising:
the filtration filter of any one of claim 1 to claim 11 welded so as to section an interior of a container made from a polymer.

13. The filter-equipped container of claim 12, wherein:

the container is configured by two opposing flexible polymer sheets of a substantially identical rectangular shape, peripheral edge portions of the respective polymer sheets being welded together;
the first weld frame of the filtration filter provided at the interior of the container is welded by a first weld portion having a substantially U-shape that is open in one direction when one of the flexible polymer sheets of the container is viewed in a thickness direction;
the second weld frame of the filtration filter is welded by a second weld portion having a substantially U-shape that is open in another direction when the other flexible polymer sheet of the container is viewed in the thickness direction; and
an opening of the first weld portion and an opening of the second weld portion face opposite sides.

14. The filter-equipped container of claim 12 or claim 13, wherein at least one of an injection port or a discharge port is provided at the container.

15. The filter-equipped container of claim 14 wherein an injection port is provided at one section of the interior of the container sectioned by the filtration filter and a discharge port is provided at another section of the interior of the container sectioned by the filtration filter.

16. A filter-equipped container comprising:

a container configured by two opposing polymer sheets, peripheral edge portions of the respective polymer sheets being welded together; and
a filter welded to an interior of the container so as to section the interior of the container and configured from a substance provided with openings and having an opening area ratio due to the openings of from 10% to 80%.

17. A method of removing foreign matter from a cell suspension, the method comprising:

injecting a cell suspension into one section of the interior of the container in the filter-equipped container of any one of claim 12 to claim 16;
passing the cell suspension through the filtration filter; and
recovering a filtrate containing cells from another section.

18. A method of removing foreign matter from a cell suspension, the method comprising:

injecting a cell suspension into one section of the interior of the container in the filter-equipped container of any one of claim 12 to claim 16;
passing the cell suspension through the filtration filter;
recovering a filtrate containing cells from another section;
injecting liquid for suspending cells into the one section;

re-suspending cells remaining in the same section;
passing the re-suspended cell suspension through the filtration filter; and
recovering a filtrate containing cells from another section.

EP 3 871 748 A1

## FIG.1

# FIG.2

FIG.3

EP 3 871 748 A1

FIG.4

EP 3 871 748 A1

## INTERNATIONAL SEARCH REPORT

| International application No. |
| --- |
| PCT/JP2019/041306 |

### A. CLASSIFICATION OF SUBJECT MATTER

B01D 39/08(2006.01)i; B01D 39/16(2006.01)i; B01D 39/20(2006.01)i; B03B 5/00(2006.01)i; B07B 1/00(2006.01)n; B07B 1/46(2006.01)n; C12M 1/00(2006.01)i; C12M 3/06(2006.01)i
FI: C12M1/00 A; B01D39/16 C; B01D39/08 Z; B01D39/20 C; B01D39/20 A; B03B5/00 Z; C12M3/06; B07B1/00 B; B07B1/46 K; B07B1/46 D
According to International Patent Classification (IPC) or to both national classification and IPC

### B. FIELDS SEARCHED

Minimum documentation searched (classification system followed by classification symbols)
B01D39/08; B01D39/16; B01D39/20; B03B5/00; B07B1/00; B07B1/46; C12M1/00; C12M3/06

Documentation searched other than minimum documentation to the extent that such documents are included in the fields searched
Published examined utility model applications of Japan 1922–1996
Published unexamined utility model applications of Japan 1971–2020
Registered utility model specifications of Japan 1996–2020
Published registered utility model applications of Japan 1994–2020

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
| --- | --- | --- |
| X | WO 2018/123619 A1 (TOYO SEIKAN GROUP HOLDINGS, LTD.) 05.07.2018 (2018-07-05) claim 1, paragraphs [0023]-[0024], fig. 1-4, claim 1, paragraphs [0023]-[0024], fig. 1-4, | 16-18 |
| A | | 1-15 |
| A | WO 2014/007382 A1 (ASAHI KASEI CHEMICALS CORP.) 09.01.2014 (2014-01-09) claims 1-2, fig. 3 | 1-18 |
| A | JP 2006-231875 A (GP DAIKYO CORP.) 07.09.2006 (2006-09-07) claim 1, paragraph [0024], fig. 1 | 1-18 |

☐ Further documents are listed in the continuation of Box C.    ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 10 January 2020 (10.01.2020) | 21 January 2020 (21.01.2020) |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (January 2015)

**INTERNATIONAL SEARCH REPORT**
Information on patent family members

International application No.

PCT/JP2019/041306

| Patent Documents referred in the Report | Publication Date | Patent Family | Publication Date |
|---|---|---|---|
| WO 2018/123619 A1 | 05 Jul. 2018 | TW 201829766 A claim 1, fig. 1-4 | |
| WO 2014/007382 A1 | 09 Jan. 2014 | US 2015/0183155 A1 claims 1-2, fig. 3 | |
| JP 2006-231875 A | 07 Sep. 2006 | US 2006/0191840 A1 claim 1, paragraph [0028], fig. 1 | |

Form PCT/ISA/210 (patent family annex) (January 2015)

**EP 3 871 748 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013114845 A
- WO 2014007382 A
- JP 2006231875 A
- JP 2018199182 A